# EUROPEAN PATENT APPLICATION

(11) **EP 4 825 385 A1**
(43) Date of publication of application: **21.10.2026**
(21) Application number: 25382399.1
(22) Date of filing: 16.04.2025
(51) Int. Cl.: A61K 35/64, A23K 10/16, A23L 33/18, A61K 38/08, A61K 38/17, A61P 29/02, C07K 7/06, C12N 15/70

(54) **BIOACTIVE PEPTIDES AND INSECT HYDROLYSATES WITH FUNCTIONAL AND THERAPEUTIC APPLICATIONS**

(71) Applicant: Insekt Label Biotech S.L., 48001 Bilbao (Bizkaia) (ES)
(72) Inventor: DEL CAMPO CASTILLO, Jose Antonio, 48001 BILBAO (BIZKAIA) (ES); ARAMBURU AMIANO, Jose Ignacio, 48001 BILBAO (BIZKAIA) (ES); OLEAGA NUÑO, Josu, 48001 BILBAO (BIZKAIA) (ES); MONTSERRAT DE LA PAZ, Sergio, 48001 BILBAO (BIZKAIA) (ES)
(74) Representative: Herrero & Asociados, S.L.

(57) **Abstract**

The present invention relates to peptides and insect hydrolysates with antiinflammatory activity, and their use in therapy.

## Description

### Technical field

The present invention belongs to the field of therapy. Specifically, it relates to peptides and insect hydrolysates with anti-inflammatory activity, and their use in therapy.

### Background of the invention

Inflammation involves the activation of the immune system in response to harmful stimuli, such as, e.g., a pathogen, infection, irritant, or damage to cells. As a stereotyped response, inflammation is a mechanism of innate immunity, as compared to adaptive immunity, which is specific for each pathogen. Inflammation can be classified as either acute or chronic.

Acute inflammation is an initial protective response of the body to remove an injurious stimulus by maintaining tissue integrity and contributing to tissue repair. The process of acute inflammation is initiated by cells already present in all tissues, mainly resident macrophages, dendritic cells, histiocytes, Kupffer cells, mastocytes, vascular endothelial cells, and vascular smooth muscle cells. At the onset of a harmful stimulus, these cells undergo activation and release inflammatory mediating and sensitizing molecules, such as, e.g., pro-inflammatory cytokines, pro-inflammatory prostaglandins, leukotrienes, histamine, serotonin, neutral proteases, bradykinin and nitric oxide. These inflammatory molecules modulate a complex series of biological events involving cellular and acellular components of the local vascular system, the immune system, and the injured tissue site to propagate and mature the inflammatory response. An acute inflammatory response requires constant stimulation to be sustained and must be actively terminated when no longer needed. Hence, acute inflammation ceases once the injurious stimulus has been removed.

However, severe or prolonged noxious stimulation results in a chronic inflammatory response that leads to a progressive shift in the type of cells present at the site of tissue injury. Chronic inflammation may be characterized as the simultaneous destruction and healing of tissue from the inflammatory process, with the net result of provoking injury rather than mediating repair. As such, chronic inflammation is a disease. As an inflammatory response can occur anywhere in the body, chronic inflammation has been implicated in the pathophysiology of a wide range of seemingly unrelated disorders which underlay a large and varied group of human diseases. For example, chronic inflammation is involved in diseases as diverse as metabolic syndrome, cardiovascular diseases, cancers, allergies, obesity, diabetes, digestive system diseases, degenerative diseases, auto-immune disorders, and Alzheimer's disease.

Despite growing knowledge about conditions that involve excessive inflammation, treatments for such conditions remain elusive. In addition, many anti-inflammatory therapies are associated with harmful side effects. Thus, there is a continuing need for the development of new and improved therapeutic options for the treatment of inflammation, and inflammatory diseases.

Interestingly, the authors of the present invention have developed new peptides with specific biological activity, particularly with anti-inflammatory properties, and have demonstrated effectiveness on targeted molecular pathways identified in both *in vitro* and *in vivo* models. Said peptides are comprised in an insect hydrolysate which also exhibits said anti-inflammatory properties.

This ability to generate peptides and hydrolysates with specific anti-inflammatory activity opens new opportunities for therapeutic applications and the development of functional products for the biomedical and food industries.

### Object of the invention

A **first aspect** of the invention relates to a peptide consisting of an amino acid sequence selected from the group consisting of SEQ ID No. 1-SEQ ID No. 8.

A **second aspect** of the invention relates to a nucleic acid encoding the peptide of the first aspect of the invention, optionally the nucleic acid is linked to a promoter sequence.

A **third aspect** of the invention relates to an expression vector comprising the nucleic acid of the second aspect of the invention.

A **fourth aspect** of the invention relates to a host cell comprising the peptide of the first aspect of the invention, the nucleic acid of the second aspect of the invention and/or the vector of the third aspect of the invention.

A **fifth aspect** of the invention relates to a hydrolysate comprising at least one of the peptides of the first aspect of the invention.

A **sixth aspect** of the invention relates to a composition comprising the peptide of the first aspect of the invention, the nucleic acid of the second aspect of the invention, the vector of the third aspect of the invention, the host cell of the fourth aspect of the invention, and/or the hydrolysate of the fifth aspect of the invention, and one or more vehicles, diluents, solvents, excipients or additives.

A **seventh aspect** of the invention relates to a medicament, food supplement, or food product characterised in that it comprises the peptide of the first aspect of the invention, the nucleic acid of the second aspect of the invention, the vector of the third aspect of the invention, the host cell of the fourth aspect of the invention, the hydrolysate of the fifth aspect of the invention and/or the composition of the sixth aspect of the invention.

An **eighth aspect** of the invention relates to the peptide of the first aspect of the invention, the nucleic acid of the second aspect of the invention, the vector of the third aspect of the invention, the host cell of the fourth aspect of the invention, the hydrolysate of the fifth aspect of the invention and/or the composition of the sixth aspect of the invention, for use in therapy or medicine.

A **ninth aspect** of the invention relates to the peptide of the first aspect of the invention, to the nucleic acid according to any one of the embodiments of the second aspect of the invention, to the vector according to any one of the embodiments of the third aspect of the invention, to the host cell according to any one of the embodiments of the fourth aspect of the invention, to the hydrolysate according to any one of the embodiments of the fifth aspect of the invention, and/or to the composition according to any one of the embodiments of the sixth aspect of the invention, for use in the treatment of inflammation or an inflammatory disease.

A **tenth aspect** of the present invention relates to a method for preparing the hydrolysate of the fifth aspect of the invention comprising the following steps:
(a) Enzymatic hydrolysis of insect pulp with one or more enzymes selected from the group of proteases, serine proteases or aminopeptidases, under conditions suitable for the enzyme(s),
(b) Inactivation of the enzyme(s).

An **eleventh aspect** of the invention relates to a hydrolysate obtainable by the method according to the tenth aspect of the invention.

A **twelfth aspect** of the present invention refers to the use of the hydrolysate of the fifth aspect or eleventh aspect of the invention for direct consumption or as an ingredient in the food or feed industry.

Other objects, features, advantages and aspects of the present application will be apparent to the person skilled in the art from the description and the attached claims.

### Detailed description of the invention

As used in the present application, the singular forms "a/an", "one" and "the" include their corresponding plurals unless the context clearly indicates otherwise. The term "and/or" means any of the items, any combination of the items, or all of the items with which this term is associated. Unless otherwise defined, all technical terms used herein have the meaning that a person ordinary skilled in the art to which this invention pertains usually understands.

In order to facilitate understanding and clarify the meaning of certain terms in the context of the present invention, the following definitions and particular and preferred embodiments thereof are provided, applicable to all embodiments of the different aspects of the present invention.

The term *"insects"* as used herein refers to insects in any development stage, such as adult insects, insect larvae, insect prepupae and pupae. The insect can be any insect of the class Insecta and arthropod phylum. In a particular embodiment, the insect is *Tenebrio molitor, Locusta migratoria, Acheta domesticus,* or *Hermetia illucens.* Preferably, as used in the Examples, the insect is *Tenebrio molitor.*

As used herein, the term "subject" refers to a mammalian subject. As such, treatment of any animal in the order mammalian is envisioned. Such animals include, but are not limited to, human; non-human primates such as chimpanzees and other apes and monkey species; farm animals, such as cattle, sheep, rabbits, pigs, goats and horses; domestic mammals such as dogs and cats; laboratory animals including rodents, such as mice, rats and guinea pigs, and the like. Thus, the present invention is contemplated for use in veterinary applications as well as human use. Preferably, the subject is a human.

"Treatment" of a subject herein refers to both therapeutic treatment and prophylactic or preventative measures. Those in need of treatment include those already with a disease or disorder as well as those in which it is to be prevented. Hence, the subject may have been diagnosed as having a disease or disorder or may be predisposed or susceptible to a disease or disorder.

The expression "effective amount" refers to an amount that is effective for preventing, ameliorating or treating a disease or disorder. Such an effective amount will generally result in an improvement in the signs, symptoms or other indicators of a disease or disorder.

A "symptom" of a disease or disorder is any morbid phenomenon or departure from the normal in structure, function, or sensation, experienced by the subject and indicative of a disease or disorder.

As used herein, "inflammatory disease" may include diseases, disorders or otherwise abnormal conditions that are associated with inflammation or have an inflammation component.

The authors of the present invention have developed and characterised a protein hydrolysate from insects and the peptides comprised in said hydrolysate. Interestingly, both the hydrolysate and the peptides show anti-inflammatory activity (see Examples 2-6), providing therapeutic options for the treatment of inflammation, and inflammatory diseases. Thus, in a **first aspect,** the present invention relates to a peptide consisting of an amino acid sequence selected from the group consisting of SEQ ID NOs: 1-SEC ID Nos: 8.

In a particular embodiment, the peptide is an isolated peptide or a pure peptide. Preferably, the peptide is at least 95% pure, or at least 99% pure, i.e. free of impurities. In a particular embodiment according to any one of the embodiments of the first aspect of the invention, the peptide has an anti-inflammatory activity capable of reducing the levels of interleukin 1β (IL-1β), interleukin 6 (IL-6), interferon γ (IFN-γ), tumor necrosis factor alpha (TNF-α), enzyme inducible nitric oxide synthase (iNOS) and/or Toll-like receptor 4 (TLR4) by, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, particularly reducing levels in comparison with an inflammation-induced state (e.g., by addition of an inducer of the inflammatory response, e.g., lipopolysaccharide, more particularly such as in section 1.2 or 1.4 of Example 1).

In another particular embodiment according to any of the embodiments of the first aspect of the invention, the peptide has an anti-inflammatory activity capable of increasing the levels of interleukin 10 (IL-10), interleukin 13 (IL-13) and/or interleukin 4 (IL-4) by, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, particularly increasing levels in comparison with an inflammation-induced state (e.g., by addition of an inducer of the inflammatory response, e.g., lipopolysaccharide, more particularly such as in section 1.2 or 1.4 of Example 1).

A **second aspect** of the invention relates to a nucleic acid encoding the peptide of the first aspect of the invention, optionally the nucleic acid is linked to a promoter sequence. Methods for defining the nucleotide sequence of the nucleic acid from the peptide sequence of the invention are well known to the person skilled in the art. Similarly, the promoter sequences are well known to the person skilled in the art.

The nucleic acid of the second aspect of the invention may be comprised in a vector. Thus, a **third aspect** of the invention relates to a vector comprising the nucleic acid of the second aspect of the invention.

The term 'vector' as used herein refers to a nucleic acid molecule capable of propagating another nucleic acid to which it has been attached. The term includes the vector as a self-replicating nucleic acid structure as well as the vector incorporated into the genome of a host cell into which it has been introduced. Certain vectors are capable of directing the expression of the nucleic acids to which they are operatively attached. Such vectors are referred to herein as 'expression vectors'. Thus, in a preferred embodiment of the invention, the vector is an expression vector.

The vector of the third aspect of the invention can be introduced into a host cell. Thus, a **fourth aspect** of the invention relates to a host cell comprising the peptide of the first aspect of the invention, the nucleic acid of the second aspect of the invention and/or the vector of the third aspect of the invention. Preferably, the host cell is a recombinant host cell.

The term 'host cell' is used interchangeably with 'host cell line' or 'host cell culture' and all refer to cells into which an exogenous nucleic acid has been introduced, including the progeny of such cells. Host cells include 'transformants' and 'transformed cells', which include primary transformed cells and progeny derived therefrom regardless of passage number. The methods of host cell transformation are well known to those skilled in the art.

The host cell can be either prokaryotic or eukaryotic. The prokaryotic host cell is preferably bacterial. Preferred eukaryotic host cells include yeast, insect and mammalian cells, preferably vertebrate cells (such as, for example, mouse, rat or monkey cells) or human cell lines (such as, for example, fibroblast or colon cells). More preferably, the host cell is a yeast, e.g. *Saccharomyces cerevisiae.*

The authors of the present invention have characterized different insect hydrolysates with anti-inflammatory activity *in vitro* and/or anti-inflammatory activity *in vivo,* which are able to induce an anti-inflammatory response (see Examples 2-5). Thus, a **fifth aspect** of the invention relates to a hydrolysate, particularly an insect hydrolysate, comprising at least one peptide according to the first aspect of the invention, so the hydrolysate may comprise a peptide of sequence SEQ ID NOs: 1-8 or combinations thereof. In a particular embodiment, the hydrolysate comprises one, several (two, three, four, five, six, seven) or all (eight) peptides of sequence selected from the group consisting of SEQ ID NO: 1 - SEQ ID NO: 8. More particularly, the hydrolysate comprises SEQ ID NO: 1 and/or SEQ ID NO: 2, and optionally one, two, three, four, five or six peptides of sequence selected from the group consisting of SEQ ID NO: 3 - SEQ ID NO: 8. In another particular embodiment, the hydrolysate of the fifth aspect of the invention comprises the peptides of SEQ ID NO: 1 and/or SEQ ID NO: 2, and SEQ ID NO: 3 and/or SEQ ID NO: 4, which are shown to have a good anti-inflammatory activity.

Preferably, the hydrolysate comprises all eight peptides of SEQ ID NOs: 1-8, as the hydrolysate of the Examples does, which has good anti-inflammatory activity *in vitro and in vivo.*

In a particular embodiment according to any one of the embodiments of the fifth aspect of the invention, the hydrolysate of the invention has an anti-inflammatory activity capable of reducing the levels of IL-1β, IL-6, IFN-γ, TNF-α, TLR4, and/or iNOS by, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, particularly reducing levels in comparison with an inflammation-induced state (e.g., by addition of an inducer of the inflammatory response, e.g., lipopolysaccharide, more particularly such as in section 1.2 or 1.4 of Example 1).

In another particular embodiment according to any one of the embodiments of the fifth aspect of the invention, the hydrolysate of the invention has an anti-inflammatory activity capable of increasing the levels of IL-10, IL-13 and/or IL-4 by, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%, particularly increasing levels in comparison with an inflammation-induced state (e.g., by addition of an inducer of the inflammatory response, e.g., lipopolysaccharide, more particularly such as in section 1.2 or 1.4 of Example 1).

The peptide, nucleic acid, vector, host cell and hydrolysate of the invention can be formulated in different types of compositions. Thus, a **sixth aspect** of the invention refers to a composition comprising, consisting of, or consisting essentially of the peptide of the first aspect of the invention, the nucleic acid of the second aspect of the invention, the vector of the third aspect of the invention, the host cell of the fourth aspect of the invention, and/or the hydrolysate of the fifth aspect of the invention, and one or more vehicles, diluents, solvents, excipients or additives.

In a particular embodiment according to any one of the preceding claims, the composition is a nutraceutical, pharmaceutical, or food composition, preferably it is a nutraceutical composition or a food composition.

The vehicles, diluents, solvents, excipients or additives are widely known to the person skilled in the art, who knows which one(s) to choose depending on the type of composition and the desired form of administration. For example, pharmaceutically acceptable excipients and salts may be those described in Remington's Pharmaceutical Sciences, 17th edition. Alfonso R. Gennaro (Ed.), Mack Publishing Company, Easton, PA, U.S.A., 1985 and more recent editions, and in the Encyclopaedia of Pharmaceutical Technology.

In a particular embodiment, the composition of the sixth aspect of the invention comprises one, several (two, three, four, five, six, seven) or all (eight) peptides of sequence selected from the group consisting of SEQ ID NO: 1 - SEQ ID NO: 8. More particularly, the composition comprises SEQ ID NO: 1 and/or SEQ ID NO: 2, and optionally one, two, three, four, five or six peptides of sequence selected from the group consisting of SEQ ID NO: 3 - SEQ ID NO: 8. In another particular embodiment, the composition comprises all eight peptides of SEQ ID NOs: 1-8.

In a preferred embodiment, the composition of the sixth aspect of the invention comprises the peptides of SEQ ID NO: 1 and/or SEQ ID NO: 2, and SEQ ID NO: 3 and/or SEQ ID NO: 4, which are shown to have good anti-inflammatory activity.

The hydrolysate and the peptide, nucleic acid, vector, host cell and composition of the invention can be formulated as medicaments, food supplements, or food products. Thus, a **seventh aspect** of the invention relates to a medicament, food supplement, or food product characterised in that it comprises the peptide of the first aspect of the invention, the nucleic acid of the second aspect of the invention, the vector of the third aspect of the invention, the host cell of the fourth aspect of the invention, the hydrolysate of the fifth aspect of the invention and/or the composition of the sixth aspect of the invention.

"Food supplement" refers to any food component that provides specific nutritional and/or functional components and does not provide the full energy value required (i.e. generally less than 2,000 or 2,500 kcal/day). They consist of concentrated sources of nutrients (e.g., vitamins, minerals) or other substances having a nutritional or physiological effect, in single or combined form, marketed in dosage form (i.e., capsules, tablets, pills, powder sachets, liquid ampoules or other similar forms), to be taken in small unit quantities. They also include dietary products, such as diet drinks. Also included are ingredients that can be added to the foodstuff before consumption or preparations that can be consumed as such.

"Food product" refers to any product, solid, gel or liquid, natural or processed, intended to be ingested by a human. It includes but is not limited to health foods and beverages, functional foods and beverages, and foods and beverages for specified health use. When such foods or beverages of the present invention are used for subjects other than humans, the terms can be used to include a feedstuff. In a preferred embodiment, the food product is a functional food, which apart from its basic nutritional role from a material and energetic point of view, is capable of providing a health benefit.

To be formulated as a medicament or food supplement, the peptide, nucleic acid, vector, host cell, hydrolysate, and/or composition of the invention, can be combined with any suitable carrier, diluent, adjuvant, excipient, etc., to obtain the medicament or supplement in the desired dosage form. Thus, they can be in any form of administration, solid, gel, or liquid, and administered by any suitable route, oral, parenteral, respiratory, rectal, or topical. Advantageously, the medicament or food/dietary supplement is administered orally. Examples of such formulations, which can be prepared using well-known methods and excipients, such as those described in, 'Remington's Pharmaceutical Sciences Handbook' Mack Pub. Co., N.Y. U.S.A., are tablets, capsules, syrups and the like for oral administration, while for parenteral administration suitable forms are, for example, sterile solutions or suspensions in acceptable liquids.

To be formulated as a food product, the peptide, nucleic acid, vector, host cell, hydrolysate, and/or composition of the invention, may be combined with any common food ingredient or added to any foodstuff. In a particular embodiment, the food product is in the form of a beverage. The term 'beverage' is intended to include liquids and syrups, as well as powder formulations to be dissolved in water or other liquid components for the preparation of instant beverages. In a particular embodiment according to any one of the embodiments of the seventh aspect of the invention, the beverage is water, a dairy beverage, an energy drink, a soft drink, or a juice.

As shown in Examples 2-5, the hydrolysate of the invention has anti-inflammatory activity *in vitro* and shows an anti-inflammatory effect in a mouse-model of acute and chronic inflammation. Furthermore, Example 6 shows that the peptides of sequence SEC ID NOs:1-4 have anti-inflammatory activity *in vitro.* Thanks to said anti-inflammatory activity, both the peptides and the hydrolysates of the invention can be used in medicine and therapy, in particular for the treatment of inflammation or an inflammatory disease. Thus, in an **eighth aspect,** the present invention relates to the peptide according to any one of the embodiments of the first aspect of the invention, to the nucleic acid according to any one of the embodiments of the second aspect of the invention, to the vector according to any one of the embodiments of the third aspect of the invention, to the host cell according to any one of the embodiments of the fourth aspect of the invention, to the hydrolysate according to any one of the embodiments of the fifth aspect of the invention, and/or the composition according to any one of the embodiments of the sixth aspect of the invention, for use in therapy or medicine.

Likewise, the eighth aspect of the invention relates to the use of the peptide according to any one of the embodiments of the first aspect of the invention, of the nucleic acid according to any one of the embodiments of the second aspect of the invention, of the vector according to any one of the embodiments of the third aspect of the invention, of the host cell according to any one of the embodiments of the fourth aspect of the invention, of the hydrolysate according to any one of the embodiments of the fifth aspect of the invention, and/or of the composition according to any one of the embodiments of the sixth aspect of the invention, for preparing a medicament, a nutraceutical composition, a functional food product, or a food supplement. In a particular embodiment, said nutraceutical composition, product or supplement can be used as an adjuvant, particularly an adjuvant in therapy or medicine.

As indicated above, the peptide and hydrolysate of the invention has anti-inflammatory activity and can be used for the treatment of inflammation or an inflammatory disease. Thus, a **ninth aspect** of the invention relates to the peptide according to any one of the embodiments of the first aspect of the invention, to the nucleic acid according to any one of the embodiments of the second aspect of the invention, to the vector according to any one of the embodiments of the third aspect of the invention, to the host cell according to any one of the embodiments of the fourth aspect of the invention, to the hydrolysate according to any one of the embodiments of the fifth aspect of the invention, and/or to the composition according to any one of the embodiments of the sixth aspect of the invention, for use in the treatment of inflammation, in particular pathological inflammation, or an inflammatory disease. The ninth aspect of the invention also relates to the peptide according to any one of the embodiments of the first aspect of the invention, to the nucleic acid according to any one of the embodiments of the second aspect of the invention, to the vector according to any one of the embodiments of the third aspect of the invention, to the host cell according to any one of the embodiments of the fourth aspect of the invention, to the hydrolysate according to any one of the embodiments of the fifth aspect of the invention, and/or to the composition according to any one of the embodiments of the sixth aspect of the invention, for use as an adjuvant in the treatment of inflammation or an inflammatory disease.

Likewise, the ninth aspect of the invention relates to the use of the peptide according to any one of the embodiments of the first aspect of the invention, of the nucleic acid according to any one of the embodiments of the second aspect of the invention, of the vector according to any one of the embodiments of the third aspect of the invention, of the host cell according to any one of the embodiments of the fourth aspect of the invention, of the hydrolysate according to any one of the embodiments of the fifth aspect of the invention, and/or of the composition according to any one of the embodiments of the sixth aspect of the invention, for the preparation of a medicament, adjuvant, nutraceutical composition, functional food product, or food supplement, for the treatment of inflammation or an inflammatory disease.

The ninth aspect of the invention also relates to a method for treating inflammation or an inflammatory disease in a subject (preferably human), comprising administering, preferably in an effective amount, to said subject the peptide according to any one of the embodiments of the first aspect of the invention, of the nucleic acid according to any one of the embodiments of the second aspect of the invention, of the vector according to any one of the embodiments of the third aspect of the invention, of the host cell according to any one of the embodiments of the fourth aspect of the invention, of the hydrolysate according to any one of the embodiments of the fifth aspect of the invention, and/or of the composition according to any one of the embodiments of the sixth aspect of the invention.

As mentioned above, inflammatory diseases may be as diverse as metabolic syndrome, cardiovascular diseases, cancers, allergies, obesity, diabetes, digestive system diseases, degenerative diseases, auto-immune disorders, and Alzheimer's disease. These diseases have an inflammation component. For example, the Nlrp3 inflammasome has recently been implicated in the development of the metabolic syndrome through the impairment of adipose tissue insulin sensitivity. Crystalline cholesterol present in atherosclerotic lesions has also been shown to be a potent activator of Nlrp3, thereby participating in the inflammatory process present in atherosclerotic plaques (Mori MA et al., Circ Res. 2011 May 13;108(10):1160-2. doi: 10.1161/RES.0b013e318220b57b. Erratum in: Circ Res. 2011 Jun 24;109(1):e13. PMID: 21566220; PMCID: PMC3660550). Moreover, neuroinflammation plays a key role in Alzheimer's disease and other neurodegenerative disorders. For example, when an inflammatory response occurs due to a failure to clear β-amyloid aggregates, it promotes the progression of neurodegenerative diseases.

Thus, in a particular embodiment of any of the embodiments of the ninth aspect of the present invention, the inflammatory disease is selected from the group consisting of metabolic syndrome, cardiovascular diseases, cancers, allergies, obesity, diabetes, digestive system diseases, degenerative diseases, auto-immune disorders, and neurodegenerative disease (e.g., Alzheimer's disease). More particularly, the inflammatory disease is selected from the group consisting of metabolic syndrome, liver steatosis, non-alcoholic fatty liver disease, osteoarthritis, systemic inflammation, cardiovascular disease, or neuroinflammation-related disease (e.g., Alzheimer's disease).

In another particular embodiment of any of the embodiments of the ninth aspect of the present invention, the inflammatory disease is a neurodegenerative disease, preferably said neurodegenerative disease is selected from the group consisting of Alzheimer's disease, Parkinson's disease, Huntington's disease, juvenile parkinsonism, dementia with Lewy bodies, multiple sclerosis, amyotrophic lateral sclerosis, frontotemporal dementia, corticobasal degeneration, chronic traumatic encephalopathy, and spinocerebellar ataxia type 3/Marchado-Joseph disease, more preferably said neurodegenerative disease is Alzheimer's disease.

In a preferred embodiment according to any one of the methods of treatment of the invention, the administration is carried out orally. The dosage will depend on various factors such as weight of the subject, sex, type and severity of the conditions to be treated, etc. and will be easily determined by the skilled practitioner.

The uses and methods described in the eighth and ninth aspects of the invention in which insect hydrolysates or compositions comprising them are used or administered are of great advantage because they are ingredients of natural origin which have no or minimal toxicity.

A **tenth aspect** of the invention relates to a method for obtaining a hydrolysate as defined in any one of the embodiments of the fifth aspect of the invention. In particular, the method comprises the following steps:
(a) Enzymatic hydrolysis of insect pulp with one or more enzymes selected from the group consisting of protease, serine protease or aminopeptidase, alcalase, trypsin, pepsin, chymotrypsin, under conditions suitable for the selected enzyme(s);
(b) Inactivation of the enzyme.

In a particular embodiment, "insect pulp" is the product obtained after mechanical size reduction of the insects to a size of less than 0.5 mm.

Preferably, the insect is *Tenebrio molitor,* which as shown in the Examples provides a highly bioactive hydrolysate.

In another particular embodiment of the tenth aspect of the invention, step a) is carried out with one or more enzymes selected from the group consisting of:
- endoprotease subtilisin type, preferably E.C. 3.4.21.14,
- protease from *Bacillus amyloliquefaciens,* preferably E.C. 3.4.24.28,
- serine protease from *Bacillus licheniformis,* preferably E.C. 3.4.21.62.

Preferably, the enzyme is an endoprotease subtilisin type E.C. 3.4.21.14, as used in the Examples, which advantageously resulted in a hydrolysate with very high anti-inflammatory activity.

As for the hydrolysis conditions, the person skilled in the art knows how to choose the conditions for each enzyme. Regarding temperature, in a particular embodiment according to any one of the preceding embodiments, the hydrolysis temperature is from 45°C to 70°C, more particularly from 50°C to 65°C. Regarding time, in a particular embodiment according to any one of the preceding embodiments, the hydrolysis is carried out from 1 h to 6 h, preferably from 1.5 h to 4 h. Regarding pH, in a particular embodiment according to any one of the preceding embodiments, the hydrolysis is carried out at a pH from 6 to 8, preferably from 6.5 to 7.5.

In another particular embodiment according to any one of the embodiments of the tenth aspect of the invention, the method further comprises the following steps:
c) phase separation; and/or
d) concentration; and/or
e) drying.

Regarding step c), in a particular embodiment according to any one of the preceding embodiments, the hydrolised product can then be fractionated by any method known by the skilled in the art. For example, it can be fractionated by any one of the following methods:
i) Screening: to separate solids bigger than 2.0 mm from the liquid.
ii) Mechanical decantation: to separate the light phase (oil) from the heavy phase (hydrolysate) and decantable solids smaller than 2.5 mm.
iii) Tangential filtration: to separate the protein hydrolysate according to the molecular weights required. A preliminary ultrafiltration can be carried out, followed by microfiltration and nanofiltration.

Regarding step d), any concentration method known by the skilled in the art can be used.

Finally, regarding step e) drying can be carried out by any method known by the skilled in the art. Preferably by spray drying or lyophilization. This step can be carried out under vacuum conditions.

In an **eleventh aspect,** the invention relates to a hydrolysate obtainable by the method according to any one of the embodiments of the tenth aspect of the invention. Likewise, it refers to said hydrolysate for use in medicine or therapy, for use in the treatment of inflammation or an inflammatory disease and for use as an adjuvant in the treatment of inflammation or an inflammatory disease. It also refers to a composition comprising said hydrolysate, and one or more vehicles, diluents, solvents, excipients or additives, preferably the composition is a nutraceutical, pharmaceutical, or food composition. Likewise, it refers to a medicament, food supplement, or food product comprising said hydrolysate. The embodiments of the fifth to ninth aspects are applicable to the eleventh aspect of the present invention.

Finally, a **twelfth aspect** of the present invention refers to the use of the hydrolysate of the fifth aspect or eleventh aspect of the invention for direct consumption or as an ingredient in the food or feed industry.

The present invention will be illustrated further by means of the following non-limiting examples.

### EXAMPLES

### Example 1. Material and Methods

### 1.1. PRODUCTION OF AN INSECT HYDROLYSATE AND ITS DIGESTED FORM

### 1.1.1. Hydrolysate preparation

Insect hydrolysates were prepared by crushing *Tenebrio molitor,* and then, the crushed *Tenebrio molitor* (insect pulp, size of less than 0.5 mm), was enzymatically hydrolised with endoprotease subtilisin type E.C. 3.4.21.14. In particular, the crushed *Tenebrio molitor* was mixed with process water in a ratio of 1:1 to 1:3. The mixture was heated to a temperature of 60°C. The pH was then adjusted to 7.25. The heated and pH-adjusted mixture was kept under stirring. When the desired temperature and pH were reached, the enzyme was added in a variable proportion, usually between 0.5% of the amount of protein contained in *Tenebrio molitor.* The hydrolysis time was 2 hours. After this time, the enzyme was inactivated by heating to 85-90 °C for 15 minutes.

The hydrolised product was then fractionated by tangential filtration. A preliminary ultrafiltration was carried out, followed by microfiltration and nanofiltration. The membrane sections are selected according to the peptides sought, using a FR (PVDF 800kDa) MAX membrane for microfiltration.

Finally, the fractionated product obtained from tangential filtration was concentrated by evaporating the water, using a vacuum evaporator.

The obtained protein hydrolysate is hereinafter referred to as TMH-IL.

### 1.1.2. Digested protein hydrolysate

To obtain the digested protein hydrolysate, a simulated gastrointestinal digestion (SGID) was performed according to the standardised INFOGEST protocol, an *in vitro* model of standard static digestion (Brodkorb, A., Egger, L., Alminger, M. et al. INFOGEST static in vitro simulation of gastrointestinal food digestion. Nat Protoc 14, 991-1014 (2019)). The aim of this SGID was to simulate the effect of digestive enzymes on the components to be analysed: α-amylase (EC 3.2.1.1), pepsin (EC 3.4.23.1), pancreatic lipase (EC 3.1.1.1.3), pancreatin and bile salts diluted in simulated salivary, gastric and intestinal fluids, respectively. To perform the digestion, 2 g of the TMH-IL were diluted 1:2 and subjected to the different phases: oral, gastric and intestinal for 0.5 h, 2-3 h and 2-3 h respectively. At the end of the SGID, enzymatic deactivation was performed by freezing at -80°C. The resulting digested hydrolysate is hereinafter referred to as dTMH-IL.

### 1.2. Bioactivity of dTMH-IL

The cells used to assess the bioactivity of dTMH-IL were human intestinal epithelial cells (CACO-2, ATCC HTB-37).

CACO-2 cells are part of the intestinal epithelial barrier and therefore have a relevant role at the biological level for the analysis of the digested sample, as this is the product that can ultimately be absorbed by CACO-2 cells once the digestion process is complete.

CACO-2 cells were maintained in high glucose Dulbecco's modified Eagle's medium supplemented with 10% heat-inactivated fetal bovine serum, 1% penicillin/streptomycin and 10% fetal bovine serum. They were incubated in a 5% CO₂ atmosphere at 37 °C and fresh medium was added every 2-3 days. Experiments were performed in 12-well plates, seeding CACO-2 cells at a density of 7.5 × 10⁵ cells per well. CACO-2 cells were pre-stimulated with lipopolysaccharide (lipopolysaccharide from *E. coli* O55:B5, Sigma-Aldrich, Cat. No. L2880, LPS hereinfater), a known inducer of the inflammatory response, at 0.5 µg/mL. After 1 h incubation, the cells were treated with dTMH-IL, for 24 h.

### 1.3. RNA isolation and real-time quantitative PCR

Total RNA was extracted from CACO-2 cells using gTZol. An A260/A280 ratio measured on a NanoDrop ND-2000 spectrophotometer was used to test the quality of the RNA. RNA (1 µg) was then reverse transcribed using the enzyme retrotranscriptase (iScript) in a thermal cycler to obtain complementary DNA (cDNA) at a final concentration of 50 ng/µL. For quantitative real-time PCR amplification (RT-qPCR), a cDNA concentration of 2 ng/µL was used, diluting the previously obtained sample 25-fold. Gene-specific mRNA levels were determined on a CFX96 system using primer pairs for any gene in addition to glyceraldehyde-3-phosphate dehydrogenase (GAPDH) and hypoxanthine phosphoribosyltransferase 1 (HPRT), which were used as constitutive genes.

### 1.4. Animal model

The experimental animals used were 7-week-old male C57BL/6J mice obtained from the University of Seville. The animal handling and experimental protocol complied with the European Community guidelines for the ethical treatment of animals (EU Directive 2010; 2010/63/EU) and was approved by the Animal Research Ethics Committee of the University of Seville (RD 53/2013). Animals were fed standard chow (SD, Teklad Global 2014, West Lafayette, IN, USA) and water *ad libitum* throughout the 4-week treatment period.

After the first week of adaptation, the animals were randomly assigned to the following experimental groups (n=12): (I) fed with Standard Diet in which the inflammatory process was induced by injection of LPS administered intraperitoneally at a dose of 0.1 mg/kg/day for 4 days prior to sacrifice (LPS, n=6) (II) fed Standard Diet supplemented with the TMH-IL prepared as in section 1.1.1 (10 mg/kg/day) and induction of the inflammatory process with LPS (TMH-IL+LPS, n=6). The insect meal was administered orally via a gastro-esophageal tube. At the same time, another group of animals (n=6) were fed a standard diet (SD), forming the control group. Animals were anaesthetised and killed at the end of week 4 by intraperitoneal injection of ketamine (80-100 mg/kg) and xylazine (10-12.5 mg/kg). After anaesthesia, the hind paws of each mouse were collected for isolation of bone marrow, brain, heart, intestine, liver and spleen, as well as fresh blood and serum for RT-qPCR and immunoassays. Faeces were collected from the animals prior to sacrifice. All mice were weighed weekly and the measurement expressed in grams, and the amount of weight gained or lost during the study was calculated in the same way. The amount of food consumed each week was also calculated and expressed in grams.

### 1.5. Bone marrow cell isolation and differentiation

To assess the bioactivity of the TMH-IL sample, bone marrow-derived macrophage cells were selected from the C57BL/6J mice chosen for the experimental animal study (see section 1.4). These progenitor cells have the ability to differentiate into macrophages after exposure to macrophage colony growth factor (M-CSF).

After the animals were sacrificed, the femur and tibia were removed from the hind legs, cleaned, the ends were cut off and 0.01 M PBS was passed through the bone with a needle over a sieve. The cells were centrifuged and then maintained for 7 days in Roswell Park Memorial Institute (RPMI) 1640 medium supplemented with heat-inactivated fetal bovine serum, 1% penicillin/streptomycin and 1% antifungal. M-CSF was then added at 20 ng/ml. They were incubated in a 5% CO₂ atmosphere at 37 °C and fresh differentiation medium was added every 2-3 days. The experiments were performed in 12-well plates and the bone marrow-derived cells were seeded at a density of 2×10⁶ cells per well.

### 1.6. Enzyme-linked immunoabsorbent assay (ELISA)

This assay was carried out using Diaclone ELISA kits (Diaclone bioNova, Ref#950.030.048), highly sensitive assays designed to allow the quantitative determination of proteins from cell culture supernatants using specific standards. This assay was carried out according to the manufacturer's instructions.

The capture antibody was first added to a 96-well plate and incubated overnight at 4 °C. The next day, it was removed, and two consecutive washes were performed before the blocking buffer was added to saturate the binding sites to avoid unspecificity and incubated for 2 h at room temperature, after which it was removed, and three consecutive washes were performed before the cell culture supernatants of bone marrow derived macrophages were added for analysis.

### Example 2. Bioactivity of dTMH-IL in CACO-2 cells

First of all, cell viability of CACO-2 cells at different concentrations of dTMHL-IL was analysed by a MTT (tetrazolium salt, 3-(4,5-dimethylthiazol-2-yl)-2,5-diphenyltetrazolium bromide) assay. CACO-2 cells were viable at all the concentrations tested (ranging from 1 µg/mL to 500 µg/mL) (data not shown).

Then, gene expression of several genes related to the inflammatory response was evaluated. The results obtained from each of the gene amplifications in their respective RT-qPCRs were normalised and standardised, first by comparison with the expression of the constitutive genes HPRT and GAPDH, and then by normalising their values with respect to those of the LPS-activated cells, to obtain relative expression values for each gene. These relative expression values are shown in Tables 1 and 2. Three pro-inflammatory genes, interleukin 1β (IL-1β), interferon γ (IFN-γ) and the enzyme inducible nitric oxide synthase (iNOS) were evaluated. IL-1β is a pro-inflammatory cytokine that plays a key role in inflammatory and autoimmune diseases. IFN-γ production after infection is the main mediator of innate and adaptive immunity and promotes macrophage activation, upregulating several pro-inflammatory parameters. The enzyme iNOs, an isoform of the enzyme nitric oxide synthase, is an important pro-inflammatory mediator with an effect on the immune system.

In Tables 1 and 2, values are shown as normalised values with respect to the control (cells pre-stimulated with LPS at 0.5 µg/mL), and presented as mean ± standard deviation (n = 3). The level of statistical significance was set at a p-value of less than 0.05..

**Table 1**

| | **IL-1β** | **IFNγ** | **iNOS** |
|---|---|---|---|
| LPS (0.5 µg/mL) | 1.00 ± 0.53 | 1.00 ± 0.40 | 1.00 ± 0.34 |
| LPS + dTMH-IL 100 µg/mL | 0.40 ± 0.01 | 0.50 ± 0.04 | 0.58 ± 0.11 |

Table 1 shows that the expression level of the three pro-inflammatory genes was reduced.

The expression of the anti-inflammatory cytokines IL-4 and IL-10 was then evaluated, which act at different levels to regulate and resolve the inflammatory response, from suppressing the development of TH1 cells (stimulating local inflammation by attracting macrophages) to inhibiting antigen presentation. Table 2 shows that the expression levels of both genes were up-regulated, at 50 µg/mL and 100 µg/mL of the digested hydrolysate used, with significant differences in the case of IL-10 when cells were in the presence of dTMH-IL at 50 µg/mL.

**Table 2**

| | **IL-4** | **IL-10** |
|---|---|---|
| LPS (0.5 µg/mL) | 1.00 ± 0.19 | 1.00 ± 0.48 |
| LPS + dTMH-IL 50 µg/mL | 3.42 ± 3.32 | 2.45 ± 0.16 |
| LPS + dTMH-IL 100 µg/mL | 2.40 ± 0.59 | 1.36 ± 0.82 |

These results suggest that the products obtained after digestion of TMH-IL have a positive effect on the immunomodulation of CACO-2 cells, acting as an immunomodulatory agent.

### Example 3. Bioactivity of TMH-IL in bone marrow-derived macrophages

Gene expression of several genes related to the inflammatory response in bone marrow cell-derived macrophages from the tibia and femur of the study mice was evaluated after induction of the inflammatory response with LPS. Macrophages are immune cells that play an essential role in inflammation, initially participating in the release of pro-inflammatory cytokines to help eliminate the pathogen (M1 phenotype) and subsequently acquiring the ability to release anti-inflammatory cytokines to suppress inflammation and promote tissue repair (M2 phenotype). The results obtained from each amplification of the target genes in their respective PCRs were normalised and standardised against the expression of the constitutive genes HPRT and β-Actin, and then, normalised against the values of mouse cells from the control group (SD).

For all the genes analysed (TNFα, IL-1β, IL-13, MRC1, TLR4), three conditions were assayed: a control (SD), a positive control (cells from mice in which the inflammatory response was induced by the injection of LPS at 0.1 mg/kg/day), and the study group of mice fed the SD diet supplemented with TMH-IL at 10 mg/kg/day and with induction of the inflammatory process with LPS. Results are shown in Table 3, represented as normalised values with respect to the control, and presented as mean ± standard deviation (n = 6). The level of statistical significance was set at a p-value of less than 0.05.

**Table 3**

| | **TNFα** | **IL-1β** | **IL-13** | **MRC1** | **TLR4** |
|---|---|---|---|---|---|
| SD | 1.00 ± 0.39 | 1.00 ± 0.16 | 1.00 ± 0.54 | 1.00 ± 0.79 | 1.00 ± 0.38 |
| LPS | 1.87 ± 1.58 | 1.43 ± 0.03 | 1.11 ± 1.31 | 1.81 ± 0.21 | 3.77 ± 0.99 |
| LPS + TMH-IL | 0.78 ± 0.70 | 0.26 ± 0.09 | 2826.48 ± 1960.15 | 1.97 ± 0.76 | 1.67 ± 0.93 |

Table 3 shows how TMH-IL produces a decrease in the expression of pro-inflammatory genes (TNF-α, IL-1β), with significant differences in the case of IL-1β, reaching even lower expression values than the control mice fed with SD.

The expression of the anti-inflammatory cytokine IL-13, which is capable of inducing macrophages to adopt an anti-inflammatory phenotype with a protective effect, was also evaluated. In this case, there was a significant increase in the expression of this immunomodulatory cytokine, with its expression being practically zero in the first two groups and then increasing significantly, indicating the anti-inflammatory effect of the TMH-IL protein hydrolysate.

MRC1 favours the stimulation of macrophages towards an M2 phenotype, with a protective effect. As shown in Table 3, there was a slight increase in the expression of this gene in mice fed with TMH-IL.

Finally, TLR4 is known to play a key role in the inflammatory response as it is the main signalling receptor for LPS, which upon binding triggers a signalling chain that culminates in the production of innate immune responses, which involves the release of pro-inflammatory cytokines. As shown in Table 3, there was a significant increase in the expression of TLR4 in cells from mice stimulated with LPS alone, due to the activation of the inflammatory response in response to LPS. In mice supplemented with TMH-IL, a significant decrease in the expression of this gene was observed, which may correlate with the potential anti-inflammatory effect of the hydrolysate.

### Example 4. Quantification of the release of cytokines involved in the inflammatory response by ELISA

To further investigate the immunomodulatory effect of TMH-IL, the release of pro- and anti-inflammatory cytokines in bone marrow cell-derived macrophages was quantified by ELISA as explained in section 1.6. The results are shown in **Table 4.**

Three conditions are reflected in Table 4: a negative control, a positive control (cells from mice injected with LPS at 0.1 mg/kg/day), study group consisting of mice fed the SD diet supplemented with TMH-IL at 10 mg/kg/day and induction of the inflammatory process with LPS. with LPS. The values obtained are standardised for each cytokine and are presented as mean ± standard deviation (n = 6). The level of statistical significance was set at a p-value of less than 0.05

**Table 4**

| | **IL-1β** | **IL-10** |
|---|---|---|
| SD | 0.00 ± 0.00 | 0.00 ± 0.00 |
| LPS | 106.21 ± 5.61 | 0.00 ± 0.00 |
| LPS + TMH-IL | 8.97 ± 17.95 | 17.72 ± 3.13 |

Consistent with the results obtained for cytokine gene expression, a significant decrease in the release of the pro-inflammatory cytokine IL-1β was observed, which almost restored the levels of the control group. Finally, the release of the anti-inflammatory cytokine, IL-10, was assessed, which went from practically zero release in the first two groups (SD and LPS) to a significant increase in macrophages derived from bone marrow cells when the mice received the LPS+TMH-IL-based diet. These results demonstrate the anti-inflammatory capacity of the *Tenebrio molitor* protein hydrolysate (TMH-IL).

### Example 5. Chronic inflammation model

During the 8-week intervention, animals were randomly assigned to the following experimental groups (n = 8-10): (I) fed SD diet and rinsed daily with mineral water; (II) fed high fat diet (HFD) and rinsed daily with mineral water; and (III) fed HFD and rinsed daily with TMH-IL (10 mg/kg/day). The hydrolysate was administered orally via a gastro-oesophageal tube.

After anaesthesia, hind paws were collected from each mouse for bone marrow isolation, liver and adipose tissue, and fresh blood and serum for RT-qPCR study. All mice were weighed weekly, and the measurement was expressed in grams and the amount of weight gained or lost was calculated in the same way. The amount of food consumed each week was also calculated in grams and the amount of weight gained or lost during the study was also calculated. The results of the body weight are shown in **Table 5** and of the liver weight in **Table 6.**

**Table 5.- Body weight in grams**

| **Day** | **SD** | **HFD** | **HFD+TMH-IL** |
|---|---|---|---|
| 0 | 26.64 ± 1.14 | 24.61 ± 1.24 | 23.70 ± 1.69 |
| 6 | 27.81 ± 1.35 | 26.51 ± 1.27 | 25.59 ± 1.62 |
| 13 | 29.09 ± 1.32 | 28.50 ± 1.42 | 26.99 ± 1.69 |
| 20 | 29.35 ± 1.21 | 30.57 ± 2.05 | 29.12 ± 1.73 |
| 27 | 30.00 ± 1.23 | 32.29 ± 2.23 | 30.22 ± 1.76 |
| 34 | 30.30 ± 0.99 | 32.63 ± 2.37 | 28.93 ± 4.64 |
| 41 | 30.73 ± 1.04 | 35.69 ± 2.46 | 33.54 ± 2.03 |
| 48 | 30.65 ± 0.95 | 35.42 ± 3.38 | 32.13 ± 2.52 |
| 56 | 30.98 ± 0.80 | 36.90 ± 3.01 | 32.73 ± 2.65 |
| 63 | 31.64 ± 0.74 | 38.40 ± 3.68 | 34.20 ± 3.06 |

As can be seen in **Table 5,** the oral administration of TMH-IL is able to restore the body weight to the one of mice fed with a control diet, thus reversing the weight gain caused by the ingestion of a high fat diet.

**Table 6**

| | **SD** | **HFD** | **HFD+TMH-IL** |
|---|---|---|---|
| Liver weight (g) | 1.09 ± 0.23 | 1.37 ± 0.21 | 1.01 ± 0.20 |

**Table 6** shows that in the HFD group, mice showed a significant increase in liver tissue weight compared to the control group. This could be related to the development of hepatic steatosis or lipid accumulation in the liver, a common condition in models of high-fat diet-induced obesity. However, mice supplemented with TMH-IL showed a significant reduction in liver weight. This reduction suggests that TMH-IL supplementation may, to some extent, modulate liver fat accumulation.

These findings support the idea that supplementation with *Tenebrio molitor* protein hydrolysate could be a promising strategy to mitigate the adverse effects of obesity, opening up new opportunities for the development of functional foods with health-promoting properties.

Finally, relative transcriptional activity in liver tissue of different genes IL-1β; IL-6 and TNF-α genes in liver tissue were analysed by RT-PCR in liver tissue. The results are shown in **Table 7.**

**Table 7**

| | **TNFα** | **IL-1β** | **IL-6** |
|---|---|---|---|
| SD | 1.00 ± 0.60 | 1.00 ± 0.25 | 1.00 ± 0.46 |
| HFD | 2.46 ± 0.84 | 4.56 ± 1.32 | 3.52 ± 0.54 |
| HFD + TMH-IL | 1.58 ± 0.56 | 2.21 ± 0.80 | 1.84 ± 0.65 |

As can be seen in Table 7, TMH-IL supplementation reduces the expression of key cytokines such as IL-1β, IL-6 and TNF-α in a significant manner. As these cytokines play a central role in the development of chronic low-grade inflammation, characteristic of metabolic syndrome and non-alcoholic fatty liver disease, these findings support that the hydrolysate of the invention has a beneficial effect in modulating obesity-associated liver inflammation. This opens up new opportunities for the development of functional foods with health-promoting properties, for example, for the treatment of metabolic syndrome and non-alcoholic fatty liver disease.

### Example 6. Synthetic peptides

### 6.1. Peptide Identification and Synthesis

Multiple peptides were identified from TMH-IL hydrolysate using liquid chromatography-mass spectrometry. Eight peptides were selected based on their predicted anti-inflammatory properties using computational analysis.

**Table 8**

| SEQ ID NO | Sequence | Anti-inflammatory^{a} |
|---|---|---|
| 1 | SRVDFHPT | 0.479 |
| 2 | RIPSVHPD | 0.481 |
| 3 | FDGIQRPL | 0.419 |
| 4 | KAPSANLPH | 0.426 |
| 5 | VHPAPAVR | 0.444 |
| 6 | SVPVIAKPR | 0.422 |
| 7 | DITDPAHPK | 0.416 |
| 8 | IAHPDHRE | 0.408 |

| | | |
|---|---|---|
| ^{a} Pre-AIP is a prediction tool for ranking potential anti-inflammatory peptides, classifying them as low (L < 0.39) to high (H > 0.47) activity; M: medium, 0.39-0.47. | | |

The eight selected peptides show medium to high potential anti-inflammatory activity. Synthetic versions of four of the gastro-resistant identified peptides (SEQ ID NO: 1-4) were obtained through solid-phase peptide synthesis to ensure purity and reproducibility.

### 6.2. Cytotoxicity

Cytotoxicity was assayed at the doses ranging from 1 to 500 µg/mL in THP-1-cells based on the reduction of a yellow tetrazolium salt, 3-(4,5-dimethylthiazol-2-yl)-2,5-diphenyltetrazolium bromide (known as MTT), to purple water-insoluble formazan crystals. The four gastro-resistant peptides showed no cytotoxicity at the tested doses (data not shown).

### 6.3. Peptide Treatment and Experimental Conditions

The synthetic peptides were dissolved in sterile phosphate-buffered saline (PBS) and prepared at the final concentration of 50 µg/mL.

### 6.4. Cell Culture and Stimulation

THP1 cells were maintained in RPMI 1640 cell culture medium supplemented with 10% heat-inactivated fetal bovine serum and 1% penicillin/streptomycin. They were incubated in a 5% CO₂ atmosphere at 37°C, with medium renewal every 2-3 days. Experiments were performed in 12-well plates, with THP1 cells grown at a density of 1×10⁶ cells per well. To induce monocyte-macrophage differentiation, THP1 cells were cultured in the presence of phorbol 12-myristate-13-acetate (PMA) at 100 nmol/L for 4 days. On day 5, THP1 cells were stimulated with LPS at a concentration of 1 µg/mL. After 1 hour, the cells were treated for 24 hours with peptides of SEQ ID NO: 1-4 at 50 µg/mL.

### 6.5. Gene Expression

Total RNA was extracted from treated and control THP-1 derived macrophages using a commercial RNA extraction kit following the manufacturer's protocol. Complementary DNA (cDNA) was synthesized using reverse transcription. Quantitative real-time PCR (qRT-PCR) was performed to assess the expression levels of the inflammatory cytokine genes IL-1β and IL-6. Gene-specific primers were designed, and amplification was carried out using a standard SYBR Green-based qPCR protocol. The relative gene expression was calculated using the 2^-ΔΔCt method, with GAPDH as the housekeeping gene.

### 6.6. Statistical Analysis

All experiments were conducted in triplicate. Data were analyzed using one-way ANOVA followed by post hoc analysis to determine statistical significance. A p-value <0.05 was considered statistically significant.

The results are shown in **Table 9,** wherein it can be seen that the four peptides of SEQ ID NO: 1-4 are able to significantly downregulate the expression of pro-inflammatory cytokines, such as IL-1β and IL-6, suggesting that these peptides have a beneficial effect in modulating inflammation.

The peptides of SEQ ID NO: 5-8 have predicted anti-inflammatory activities in the range of or like the ones tested *in vitro,* suggesting the same ability to downregulate the expression of pro-inflammatory cytokines.

**Table 9**

| | **IL-1β** | **IL-6** |
|---|---|---|
| SD | 1.00 ± 0.01 | |
| LPS | 9.91 ± 1.24 | |
| LPS + SEQ ID NO 1 | 3.47 ± 0.20 | 2.68 ± 1.31 |
| LPS + SEQ ID NO 2 | 4.60 ± 0.57 | 2.98 ± 0.45 |
| LPS + SEQ ID NO 3 | 5.92 ± 1.91 | 3.55 ± 1.47 |
| LPS + SEQ ID NO 4 | 6.48 ± 1.01 | 2.26 ± 0.60 |

## Claims

1. Peptide consisting of an amino acid sequence selected from the group consisting of SEQ ID NO: 1-SEQ ID NO: 8, for use in therapy.

2. Peptide consisting of an amino acid sequence selected from the group consisting of SEQ ID NO: 1-SEQ ID NO: 8, for use in the treatment of inflammation or an inflammatory disease.

3. Peptide consisting of an amino acid sequence selected from the group consisting of SEQ ID NO: 1-SEQ ID NO: 8.

4. Nucleic acid encoding a peptide according to claim 3, optionally linked to a heterologous promoter sequence.

5. Expression vector comprising the nucleic acid according to claim 4.

6. Host cell comprising the peptide according to claim 3, the nucleic acid according to claim 4, and/or the expression vector according to claim 5.

7. Insect hydrolysate comprising at least one peptide according to claim 3, preferably comprising all the peptides according to claim 3.

8. Composition comprising the peptide of claim 3, the nucleic acid of claim 4, the expression vector of claim 5, the host cell of claim 6 and/or the insect hydrolysate of claim 7, and one or more vehicles, diluents, solvents or additives.

9. Nucleic acid according to claim 4, vector according to claim 5, host cell according to claim 6, insect hydrolysate according to claim 7, or composition according to claim 8, for use in therapy.

10. Nucleic acid according to claim 4, vector according to claim 5, host cell according to claim 6, insect hydrolysate according to claim 7, or composition according to claim 8, for use in the treatment of inflammation or an inflammatory disease.

11. Peptide according to claim 3, nucleic acid according to claim 4, vector according to claim 5, host cell according to claim 6, insect hydrolysate according to claim 7, or composition according to claim 8, for use as a adjuvant in the treatment of inflammation or an inflammatory disease.

12. Peptide for use according to claim 2 or 11, nucleic acid, vector, host cell, insect hydrolysate, or composition for use according to claim 10 or 11, wherein the inflammatory disease is selected from metabolic syndrome, non-alcoholic fatty liver disease, hepatic steatosis, cardiovascular diseases, cancers, allergies, obesity, diabetes, digestive system diseases, degenerative diseases, auto-immune disorders, and Alzheimer's disease.

13. Food product, medicament or food supplement, comprising the peptide of claim 3, the nucleic acid of claim 4, the expression vector of claim 5, the host cell of claim 6, the insect hydrolysate of claim 7, and/or the composition of claim 8.

14. Method for obtaining a hydrolysate according to claim 7, **characterized in that** it comprises the following steps:
a) Enzymatic hydrolysis of insect pulp with one or more enzymes selected from the group consisting of protease, serine protease or aminopeptidase, alcalase, trypsin, pepsin, chymotrypsin, under conditions suitable for the enzyme/s, preferably the enzyme is an endoprotease subtilisin type;
b) Inactivation of the enzyme.

15. Use of the hydrolysate of claim 7 or of the hydrolysate obtainable by the method of claim 14, for direct consumption or as an ingredient in the food or feed industry.
